# EUROPÄISCHE PATENTANMELDUNG

(11) **EP 0 847 744 A2**
(43) Veröffentlichungstag der Anmeldung: **17.06.1998**
(21) Anmeldenummer: 97121231.1
(22) Anmeldetag: 03.12.1997
(51) Int. Cl.: A61J 17/00

(54) **Beruhigungssauger**

(30) Priorität: 14.12.1996 DE 19652118
(71) Anmelder: Hönig, Oliver, Dr. med., 71554 Weissach im Tal (DE)
(72) Erfinder: Hönig, Oliver, Dr. med., 71554 Weissach im Tal (DE)
(74) Vertreter: Wilhelm & Dauster Patentanwälte European Patent Attorneys

(57) **Zusammenfassung**

Die Erfindung bezieht sich auf einen Beruhigungssauger mit einem Mundschild (1) und einem sich vom Mundschild nach vorn erstreckenden Mundstück (3).

Erfindungsgemäß beinhaltet der Beruhigungssauger einen biegsam und kaudruckresistent ausgelegten Kanal (6), der das das Mundstück und den Mundschild durchquert und den Außenbereich vor dem Mundstück mit dem Außenbereich hinter dem Mundschild verbindet.

Verwendung z.B. als Narkoseschnuller.

## Beschreibung

Die Erfindung bezieht sich auf einen Beruhigungssauger mit einem Mundschild und einem sich vom Mundschild nach vorn erstreckenden Mundstück. Solche Beruhigungssauger, auch Schnuller genannt, werden üblicherweise zur Beruhigung von Kleinkindern verwendet.

In der Offenlegungsschrift WO 96/19942 wird ein Entwöhnungssauger beschrieben, der einen geradlinig und im wesentlichen senkrecht zur Ebene eines Mundschildes verlaufenden Luftkanal aufweist, welcher ein ansonsten massiv aus Kautschuk oder einem ähnlichen Material bestehendes Mundstück mittig durchquert, das sich vom Mundschild nach vorn erstreckt. Entsprechend seinem Einsatzzweck als Entwöhnungssauger ist das Material des Mundstücks nicht auf Kaudruckresistenz ausgelegt, sondern besitzt die bei herkömmlichen Beruhigungssaugern übliche, relativ geringe Biegefestigkeit. Ein zeitweiliges Verschließen des Luftkanals unter Kaudruckeinwirkung ist für diesen Einsatzzweck unschädlich. Der Luftkanal mündet vorne am Mundstückvorderende aus, während er an seinem hinteren Ende in einen seitlich zum Außenbereich hinter dem Mundschild hin offenen Durchgang eines Stopfens mündet, mit welchem das Mundstück am Mundschild gehalten wird. Dabei ist das hintere Ende des Luftkanals vorzugsweise anfangs verschlossen, und der Durchtrittsquerschnitt des Kanals wird dann mittels Durchbrechen des Kanalverschlusses unter Verwendung eines geeignet über den seitlichen Stopfendurchgang eingebrachten Werkzeugs nach und nach größer gemacht. Alternativ kann ein Satz von Entwöhnungssaugern mit unterschiedlichem Luftkanaldurchtrittsquerschnitt bereitgestellt werden.

Aus der Patentschrift US 1.518.823 ist ein sowohl als Flaschensauger wie auch als Beruhigungssauger verwendbarer Sauger bekannt, bei dem ein Mundstück vorgesehen ist, das aus einem nicht biegsamen Material, z.B. aus Knochenmaterial, besteht und das von einem mittigen Längskanal durchquert wird. Im hinteren Bereich besitzt das Mundstück ein Außengewinde, mit dem es entweder in einen zugehörigen Adapter zur Verwendung als Flaschensauger oder in einen hülsenförmigen Mundschild zur Verwendung als Beruhigungssauger eingeschraubt werden kann. Der Mundschild ist dabei geschlossen ausgebildet und verschließt den Kanal nach Einschrauben des Mundstücks, so daß dem Kanal beim Einsatz des Saugers als Beruhigungssauger keine Funktion zukommt.

In der Patentschrift DE 488 ist ein Flaschensauger offenbart, bei dem ein Mundstück aus Gummi von einem nicht biegsamen Rohr aus Elfenbein oder versilbertem Metall unter Belassung eines Luftkissens durchdrungen wird.

Neben den oben erwähnten Einsatzzwecken sind auch bereits Beruhigungssauger für spezielle medizinische Zwecke vorgeschlagen worden. So ist im Gebrauchsmuster DE 90 06 946 U1 ein Schlafinhalationssauger offenbart, bei dem am Mundschild auf der dem Mundstück abgewandten Seite eine perforierte Halbkugel angebracht ist, in der sich ein Wattebausch befindet, der mit ätherischen Ölen getränkt werden kann. Die ätherischen Öle können dann durch die Perforation nach außen abgegeben und so von einem an diesem Sauger saugenden Kleinkind inhaliert werden, um beispielsweise Erkältungsbeschwerden zu lindern. In der Offenlegungsschrift EP 0 681 824 A1 ist ein weiterer medizinischer Sauger beschrieben, der einen topfartigen Aufnahmebehälter für ein Medikament an der dem Mundstück abgewandten Seite des Mundschildes aufweist. Bei diesem Sauger ist der Aufnahmebehälter jedoch nach außen abgedichtet und steht über einen durch den Mundschild hindurchführenden Durchflußkanal mit dem Inneren des Mundstücks in Fluidverbindung. Durch eine im Vorderbereich des Mundstücks eingebrachte Saugöffnung kann von einem am Sauger saugenden Kleinkind ein flüssiges bzw. verflüssigtes Medikament in den Mund eingesaugt werden. Am Aufnahmebehälter kann ein Anschlußstutzen angeformt sein, der mit einem externen Schlauch oder dergleichen verbindbar ist, um den Aufnahmebehälter kontinuierlich mit größeren Medikamentenmengen oder Flüssigkeitsmengen zu befüllen. Wenn das Medikament mit Druck in den Mundraum des Kleinkindes befördert werden soll, kann hierzu innerhalb des Aufnahmebehälters ein Blasebalg, ein Kolben oder dergleichen vorgesehen werden. Wenn der Anschlußstutzen nicht gebraucht wird, wird er durch geeignete Hilfsmittel nach außen abgedichtet.

Bei Narkosen zu operativen Eingriffen oder Untersuchungen sind Kleinkinder trotz Prämedikation und Anwesenheit eines Elternteils oft unruhig und agitiert. Um die notwendige Maskenbeatmung bereits zur Einleitung der Narkose oder nach intravenöser Gabe von Narkosemedikamenten gewährleisten zu können, muß den Kindern in dieser für sie befremdlichen Situation auch noch ihr ihnen vertrauter Beruhigungssauger genommen werden, was den Unruhezustand der Kinder zusätzlich verstärkt. Entsprechendes gilt am Ende der Narkose. Um freie Atemwege und das Ablaufen von Sekreten, wie Schleim, Speichel oder Blut z.B. während Hals-/Nasen-/Ohren-(HNO)-Operationen zu gewährleisten, sind herkömmliche Beruhigungssauger zur Beruhigung der Kinder nicht anwendbar. Andererseits kann Unruhe und Agitiertheit der Kinder in dieser Phase sogar zu Gefährdungen, wie Nachblutungen, führen.

Der Erfindung liegt als technisches Problem die Bereitstellung eines Beruhigungssaugers der eingangs genannten Art zugrunde, der sich in neuartiger Weise als medizinischer Sauger verwenden läßt und sich insbesondere als Narkoseschnuller zur Beruhigung von Kleinkindern bei Narkosen eignet.

Die Erfindung löst dieses Problem durch die Bereitstellung eines Beruhigungssaugers mit den Merkmalen des Anspruchs 1. Dieser Beruhigungssauger beinhaltet einen das Mundstück und den Mundschild durchquerenden Kanal, der den Außenbereich vor dem Mundstück mit dem Außenbereich hinter dem Mundschild verbindet.

Die dadurch gegebene Verbindung des Bereichs vor dem Mundstück mit dem Bereich hinter dem Mundschild kann für verschiedene Zwecke, insbesondere für diverse medizinische Maßnahmen, genutzt werden. So kann der Sauger als Narkosehilfsmittel während der Narkoseeinleitung und/oder -ausleitung benutzt werden. In diesem Fall kann durch den Kanal hindurch einerseits Sekret, wie Speichel oder Blut, aus dem Mund abgesaugt werden, ohne daß das Kind diese Maßnahme durch Zubeißen verhindern kann, und andererseits kann durch diesen Kanal eine behinderte oder unzureichende Atmung des Kindes verbessert werden. Insbesondere kann durch diesen Kanal hindurch unter Zuhilfenahme eines Beatmungsbeutels auch beatmet werden, entweder durch direktes Aufsetzen des Beutels oder durch Verwenden eines Adapters, der den Rohrquerschnitt des Beatmungsbeutels an den Querschnitt des Saugerkanals anpaßt. Ebenfalls erleichtert werden kann durch diesen Beruhigungssauger eine Inhalationstherapie spontan atmender Kinder, z.B. bei Asthma oder Lungenerkrankungen.

Charakteristischerweise ist der Kanal von einem biegsamen, kaudruckresistenten Material umgeben. Der Kanal kann sich auf diese Weise zum einen aufgrund seiner Biegsamkeit dem Mund des Kindes gut anpassen, ohne daß er andererseits vom Kind durch Kaudruck verschlossen oder zerbissen werden kann. Hierfür geeignete Materialien sind dem Fachmann bekannt, z.B. entsprechende Kunststoffmaterialien.

Bei einem nach Anspruch 2 weitergebildeten Sauger besteht der Kanal aus mehreren parallelen Einzelkanälen, die z.B. durch ein Mehrkanalrohr gebildet sein können. Dadurch sind mehrere voneinander getrennte Verbindungskanäle zwischen dem Außenbereich vor dem Mundstück und dem Außenbereich hinter dem Mundschild geschaffen, was sich beispielsweise zum Absaugen von Sekret durch einen Einzelkanal ohne Unterbrechung eines durch einen anderen Einzelkanal eingeströmten Luft- oder Sauerstoffstroms im Umfeld einer medizinischen Behandlung ausnutzen läßt.

Bei einem nach Anspruch 3 weitergebildeten Sauger mündet der Kanal unterhalb einer Längsmittelebene des Mundstücks aus demselben aus, wodurch besonders zuverlässig verhindert wird, daß im Gebrauch die Kanalmündung am Mundstück durch das Gaumensegel bzw. das Zäpfchen des Kindes verschlossen wird, so daß ein freier Zugang zum hinteren Mund-/Rachenraum gewährleistet ist. Vorzugsweise verläuft der Kanal hierbei in einem geradlinigen oder gebogenen Verlauf vom Mundschild aus bis zur Ausmündung im Mundstückvorderbereich in Gebrauchslage des Saugers schräg nach unten.

Bei einem nach Anspruch 4 weitergebildeten Sauger ist die hintere, zum Außenbereich hinter dem Mundschild führende Kanalmündung trichterförmig gestaltet. Dadurch kann einfach und rasch ein Absaugkatheter oder ein Beatmungsadapter während einer Narkose angesetzt bzw. eingebracht werden.

Bei einem nach Anspruch 5 weitergebildeten Sauger ist ein abnehmbares Verschlußmittel vorgesehen, um den Kanal bei Bedarf an seinem in den Außenbereich hinter dem Mundschild mündenden Ende verschließen und wieder öffnen zu können. Im verschlossenen Zustand fungiert er als normaler Schnuller, so daß sich die Kinder z.B. vor einer Narkose an ihn gewöhnen können.

Während der Narkose kann dann das Verschlußmittel je nach Bedarf entfernt und wieder angebracht werden. Besonders vorteilhaft ist die Verwendung eines Obturators, der den Kanal auf seiner ganzen Länge verschließt und dadurch ein Verschmutzen des Kanals weitestgehend verhindert.

Bei einem nach Anspruch 6 weitergebildeten Sauger ist wenigstens eine seitliche Aussparung im Mundschild vorgesehen, die als Schlauch- oder Rohrfixierung fungiert. Dadurch können z.B. Beatmungsschläuche am Mundschild fixiert werden.

Bei einem nach Anspruch 7 weitergebildeten Sauger ist der Kanal von einem separat im Mundstück vorgesehenen ein- oder mehrkanaligen Rohr gebildet, wobei die Materialien und die Materialstärken für das Rohr einerseits und das Mundstück andererseits so gewählt sind, daß sich für das Rohr eine höhere Biegesteifigkeit ergibt als für das Mundstück.

Vorteilhafte Ausführungsformen der Erfindung sind in den Zeichnungen dargestellt und werden nachfolgend beschrieben. Hierbei zeigen:
- Fig. 1: eine Längsschnittansicht eines als Narkoseschnuller verwendbaren Beruhigungssaugers,
- Fig. 2: eine Schnittansicht längs der Linie II-II von Fig. 1,
- Fig. 3: einen Querschnitt durch ein Rohr, das für einen Sauger der in den Fig. 1 und 2 gezeigten Art anstelle des dort gezeigten Rohres verwendbar ist,
- Fig. 4: eine Längsschnittansicht eines gegenüber demjenigen der Fig. 1 und 2 modifizierten, als Narkoseschnuller verwendbaren Beruhigungssaugers,
- Fig. 5: eine Draufsicht auf den Sauger von Fig. 4 längs des Pfeils D und
- Fig. 6: eine Längsschnittansicht eines weiteren gegenüber demjenigen der Fig. 1 und 2 modifizierten, als Narkoseschnuller verwendbaren Beruhigungssaugers.

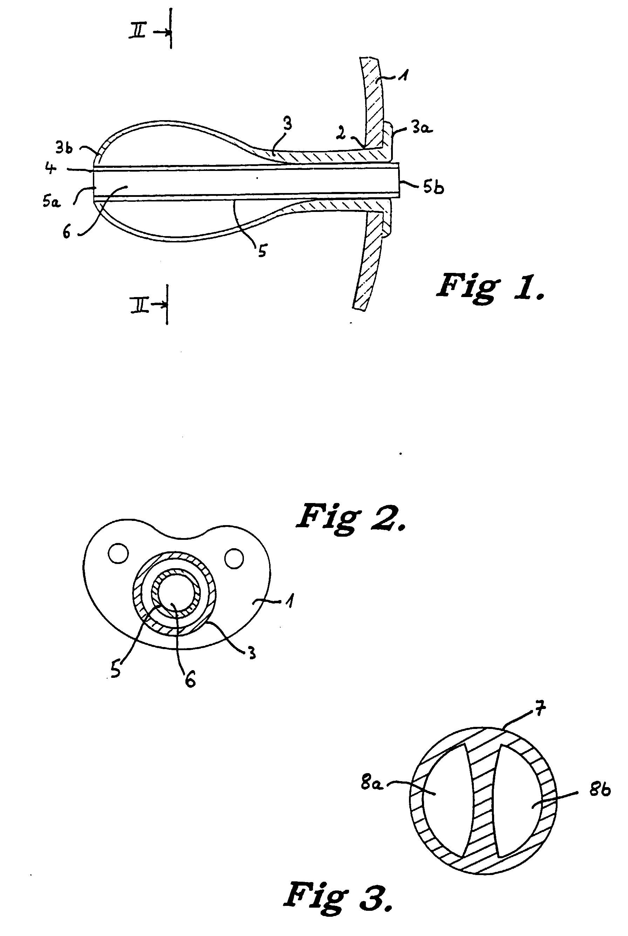

Der in den Fig. 1 und 2 dargestellte Beruhigungssauger beinhaltet einen Mundschild 1, der eine mittige Öffnung 2 aufweist, in die ein Mundstück 3 mit seinem hinteren Ende in herkömmlicher Weise eingesetzt und am Öffnungsrand des Mundschildes 1 fixiert ist. Dabei hintergreift das Mundstück 3 mit einem radialen Ringflansch 3a den Öffnungsrand des Mundschildes 1. Vom Mundschild 1 aus erstreckt sich das in einer herkömmlichen Außenkontur geformte Mundstück 3 nach vorn, wo es mit einem gerundeten Vorderende 3b abschließt, in den eine beispielsweise kreisrunde Öffnung 4 eingebracht ist.

Charakteristischerweise beinhaltet der in den Fig. 1 und 2 gezeigte Beruhigungssauger ein sich längs durch das Mundstück 3 hindurch erstreckendes Rohr 5, das mit seinem Vorderende 5a im wesentlichen bündig in der Öffnung 4 am Vorderende 3b des Mundstücks 3 liegt. Das Rohr 5 durchquert mittig die Öffnung 2 im Mundschild 1, wobei es vom hinteren Bereich des Mundstücks 3 enganliegend und damit abdichtend umschlossen und dadurch auch geführt wird. Das Rohr 5 mündet mit seinem hinteren Ende 5b in den Bereich hinter dem Mundschild 1 aus.

Durch das Rohr 5 ist somit ein durchgängiger Verbindungskanal 6 geschaffen, der den Außenbereich vor dem Mundstück 3, der im Gebrauch des Saugers dem Mundraum eines Kleinkindes entspricht, mit dem Außenbereich hinter dem Mundschild 1 verbindet, wobei dieser Außenbereich für Eingriffe durch eine Betreuungsperson auch im Gebrauch des Saugers zugänglich ist. Dadurch ist der Sauger insbesondere als Narkosehilfsmittel bei der Narkoseeinleitung und -ausleitung, d.h. als Narkoseschnuller, verwendbar. Das Rohr 5 ist hierzu durch Wahl eines geeigneten Materials und einer geeigneten Rohrstärke so beschaffen, daß es einerseits eine gewisse Biegesteifigkeit besitzt, die höher ist als das aus herkömmlichem Latex- oder Silikonmaterial gefertigte Mundstück 3, andererseits jedoch noch ausreichend biegsam ist, so daß es zum einen nicht von dem Kind durch Kaudruck verschlossen oder zerbissen werden kann und sich zum anderen dem Mund des Kindes ausreichend anpaßt.

Für die Narkoseeinleitung kann das Kind den Sauger gefahrlos über das Einschlafen hinaus behalten und ist dadurch viel ruhiger und angstfreier als ohne Schnuller. Zudem ist durch den vom Rohr 5 geschaffenen Verbindungskanal 6 hindurch eine assistierende Beatmung bei der Narkoseeinleitung möglich, ohne daß die Gefahr pharyngealer Würgereflexe besteht. Die Beatmung kann z.B. mittels eines Beatmungsbeutels erfolgen, der direkt oder über einen querschnittsanpassenden Adapter an das hintere Rohrende 5b angeschlossen wird. Je nach Bedarf können durch den vom Rohr 5 gebildeten Verbindungskanal 6 hindurch weitere medizinische Maßnahmen während der Einleitung und Ausleitung der Narkose ausgeführt werden, wie das Absaugen von Sekret, z.B. Speichel oder Blut, aus dem Mundraum des Kindes, ohne daß dies von Zubeißreflexen des Kindes erschwert wird. Nach Ende der Narkose erwacht das Kind dann wieder in vertrauter Weise mit dem Sauger und ist dadurch ruhiger und kooperativer als bei fehlendem Schnuller. Auch in dieser Phase kann je nach Bedarf über den Verbindungskanal 6 Sauerstoff verabreicht oder Sekret, wie Blut oder Speichel, abgesaugt werden. Insbesondere können damit die speziell nach HNO-Eingriffen gefürchteten Erregungszustände der Kinder gemildert werden.

In einer vorteilhaften Variante des Saugers der Fig. 1 und 2 kann anstelle des dortigen einkanaligen Rohres 5 ein Zweikanalrohr 7 vorgesehen sein, wie es in Fig. 3 im Querschnitt dargestellt ist. Dieses Rohr 7 beinhaltet zwei voneinander getrennte, parallele Kanäle 8a, 8b. Bei Einsatz dieses Zweikanalrohres 7 als das den Außenraum vor dem Mundstück 3 mit dem Außenraum hinter dem Mundschild 1 verbindende Rohr des erfindungsgemäßen Saugers stehen zwei getrennte Verbindungskanäle zur Verfügung, über die zwei der oben erwähnten Eingriffe parallel vorgenommen werden können. Beispielsweise kann bei der Narkoseeinleitung oder -ausleitung durch den einen Verbindungskanal ein Luft- oder Sauerstoffstrom zur Beatmung eingeströmt werden, während gleichzeitig über den anderen Verbindungskanal Sekret aus dem Mundraum abgesaugt werden kann.

Es versteht sich, daß neben den gezeigten und beschriebenen Beispielen und Verwendungsmöglichkeiten weitere Variationen und Einsatzzwecke für den erfindungsgemäßen Sauger möglich sind. So kann der gezeigte Sauger in seinem konstruktiven Aufbau in jeweils passender Weise modifiziert sein, insbesondere hinsichtlich der Länge, der Biegung und der Ausformung des Mundstücks, des Mundschildes und des Rohres sowie des Rohr- und damit des Verbindungskanalquerschnitts, wobei ein möglichst großer, vom Kind noch akzeptierter Querschnitt anzustreben ist, und hinsichtlich der Saugergröße insgesamt. Die letztgenannte Maßnahme betrifft insbesondere die Herstellung des Saugers in verschiedenen, den jeweiligen Altersstufen des Kindes angepaßten Größen. Anstelle der beschriebenen Einbringung eines separaten Rohres in das Mundstück kann ein durchgehender Kanal auch in Form eines Bohrungskanals durch ein im wesentlich massiv gestaltetes Mundstück hindurch realisiert sein. Das Mundstück ist dann hinsichtlich Materialart und -stärke speziell so gefertigt, daß einerseits eine ausreichende Biegsamkeit und andererseits die geforderte Kaudruckfestigkeit für den Kanal gegeben ist. Letztere gewährleistet, daß der Kanal im Gebrauch des Saugers durch den ausgeübten Kaudruck nicht verschlossen wird, sondern gut durchgängig bleibt. Des weiteren sind zahlreiche Variationen hinsichtlich der verwendeten Materialien für die verschiedenen Saugerteile möglich, beispielsweise hypallergene, latexfreie Ausführungen für speziell disponierte Kinder oder aromatisierte Ausführungen zur weiteren Erhöhung der Akzeptanz des Saugers durch die Kinder. Dazu trägt gegebenenfalls auch eine anatomisch angepaßte, gekrümmte Ausführung des Rohres bei. Dabei läßt sich der Sauger als Mehrweg- oder Einmalartikel ausführen.

Neben der Verwendung als Narkosehilfsmittel kommt ein Einsatz des erfindungsgemäßen Saugers für viele weitere medizinische Anwendungen in Betracht. In der Notfallmedizin kann er zur assistierten Beatmung und/oder Absaugung von Sekreten und/oder Sauerstoffgabe bei sehr aufgeregten Kindern dienen. In der Pädiatrie ist er zur Erleichterung der Atmung einsetzbar, beispielsweise dadurch, daß durch den vom Rohr gebildeten Verbindungskanal eine Inhalation vernebelter Medikamente bei Kindern mit Asthma oder Lungenerkrankungen vorgenommen wird. In der pädiatrischen Intensivmedizin und in der Neonatologie kann er zur Erleichterung assistierender Beatmungsformen z.B. in der Phase nach einer Beatmungstherapie eingesetzt werden. In der HNO-Heilkunde ist er zum Vermeiden des Schnarchens bei schnarchenden Kindern und/oder für Kinder mit großen Adenoiden verwendbar. Des weiteren kann der Einsatz des erfindungsgemäßen Saugers bei Kindern mit Atembehinderungen, z.B. wegen Mißbildungen, Makroglossie oder im Anschluß an eine Operation, zweckmäßig sein.

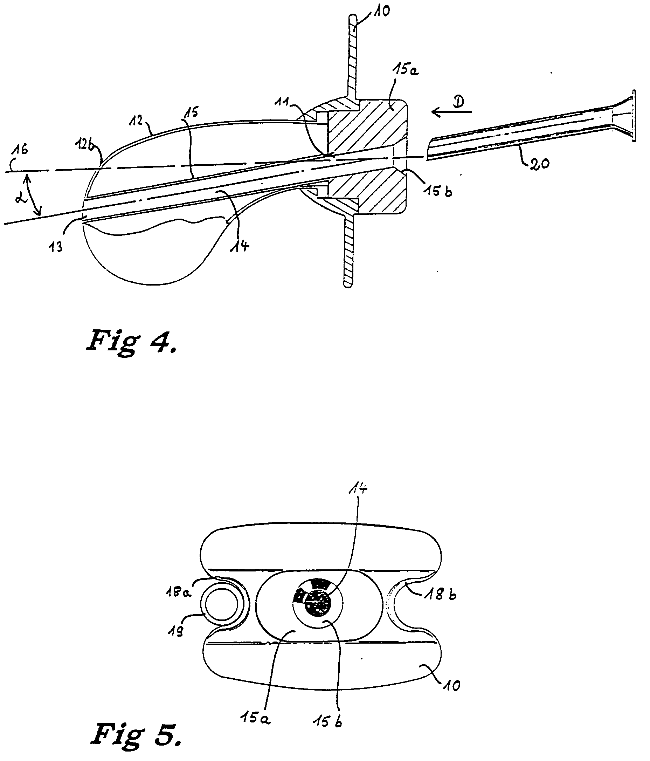

In den Fig. 4 und 5 ist eine Variante des Saugers der Fig. 1 und 2 dargestellt, die ebenfalls die zu diesem Sauger oben beschriebenen Funktionen und damit erreichbaren Vorteile sowie einige weitere Funktionalitäten aufweist, auf die nachfolgend näher eingegangen wird.

Der Sauger gemäß den Fig. 4 und 5 beinhaltet einen Mundschild 10, an dem ein Mundstück 12 eingesetzt und am Öffnungsrand des Mundschildes 10 fixiert ist, das sich von dort in einer herkömmlichen Außenkontur nach vorn erstreckt, wo es mit einem gerundeten Vorderende 12b abschließt, in den eine beispielsweise kreisrunde Öffnung 13 eingebracht ist. An dieser Öffnung 13 mündet ein Rohrkanal 14 mundstückseitig aus, der sich durch das hohle Mundstück 12 sowie durch den Mundschild 10 hindurch in den Außenbereich hinter dem Mundschild 10 erstreckt. Der Kanal 14 wird von einem Rohrstück 15 definiert, das durch eine mittige Öffnung 11 im Mundschild 10 hindurchgeführt ist und mit einem radial abkragenden Endflansch 15a fest von außen gegen den Mundschild 10 anliegt.

Im Unterschied zum Sauger der Fig. 1 bis 3 verläuft der Kanal 14 beim Sauger der Fig. 4 und 5 zur Mundstücköffnung 13 hin bezüglich der Mundschildebene schräg nach unten geneigt, wodurch die Mundstücköffnung 13 unterhalb einer gestrichelt angedeuteten Längsmittelebene 16 liegt, die mit Ausnahme dieses schrägen Kanalverlaufs eine Symmetrieebene des Saugers bildet. Dies hat den Vorteil, daß ein Verschließen der Öffnung 13 durch das Gaumensegel bzw. das Zäpfchen des Kindes beim Gebrauch des Saugers zuverlässig vermieden wird. Dadurch besteht mittels des Rohrkanals 14 stets ein freier Zugang zum hinteren Mund-/Rachenraum, über den z.B. während einer Narkose abgesaugt oder insuffliert werden kann. Der zugehörige Schrägwinkel α kann an den jeweiligen Anwendungsfall angepaßt beliebig gewählt werden, z.B. im Bereich zwischen 15° und 30°.

Das den Kanal 14 umgebende Rohr 15 ist wiederum so ausgelegt, daß es zum einen biegsam ist, damit es sich beim Gebrauch des Schnullers zusammen mit dem Mundstück 12 an den jeweiligen Mundraum ausreichend anpassen kann, zum anderen aber noch eine so hohe Biegefestigkeit aufweist, daß die gewünschte Kaudruckresistenz gegeben ist, durch die verhindert wird, daß es zu einem Verschluß des Luftkanals 14 kommt. Das Rohr 15 besitzt zu diesem Zweck eine höhere Biegesteifigkeit als die Wandung des Mundstücks 12. Alternativ kann ein massives Mundstück aus einem oder mehreren schichtartig aufeinanderfolgenden Materialien mit jeweils geeigneter Biegefähigkeit vorgesehen sein, in welches dann der durchgehende Kanal in Form einer schräg verlaufenden Bohrung eingebracht ist.

Das Rohr 15 ist an seinem mundschildseitigen Ende, an welchem der radiale Flansch 15a abkragt, mit einer trichterförmigen Aufweitung 15b versehen. Die dadurch gebildete trichterförmige Anschlußöffnung des Kanals 14 erleichtert das Anschließen bzw. Einführen von Adapterelementen, wie eines in der Medizin gebräuchlichen Normkonnektors. Das jeweilige Adapterelement wird beim Ansetzen vom Trichterrand selbsttätig in den Kanal 14 geführt, so daß ein genau zentriertes Ansetzen nicht erforderlich ist. Auf diese Weise kann z.B. ein Absaugkatheter oder ein Beatmungsadapter zur Beatmung oder zur Inhalationstherapie spontan atmender Kinder in der Intensivmedizin einfach und schnell an den Sauger angeschlossen werden.

Wie in Fig. 5 zu erkennen, besitzt der Mundschild 10 zwei V- oder U-förmige, seitliche Aussparungen 18a, 18b. Diese Aussparungen 18a, 18b können dazu verwendet werden, einen Schlauch oder ein Rohr aufzunehmen bzw. zu fixieren, z.B. einen Beatmungsschlauch 19, d.h. einen Tubus, wie für die in Fig. 5 linke Aussparung 18a gezeigt. Dazu ist die Form der Aussparungen 18a,18b der Querschnittsform des aufzunehmenden Schlauches bzw. Rohres angepaßt. Der Tubus 19 kann dann beispielsweise mittels Pflasterstreifen oder dergleichen am Mundschild 10 fixiert werden. Die seitlichen Aussparungen 18a, 18b bieten überdies eine einfache Zugangsmöglichkeit zum Absaugen der vorderen Mundhöhle.

Von Vorteil ist des weiteren eine dichte Wiederverschließbarkeit des Kanals 14 durch ein geeignetes abnehmbares Verschlußmittel, z.B. eine Folie, ein Stopfen oder einen Obturator 20, wie er in Fig. 4 in abgenommener Lage gezeigt ist. Ein solcher Obturator 20 entspricht in Form und Abmessung dem zu verschließenden Kanal 14, so daß er diesen nach Einsetzen in denselben vollständig bis nach vorn zur Mundstücköffnung 13 verschließt. Damit kann der Sauger immer dann, wenn der Durchlaßkanal 14 nicht benötigt wird, in seinem mit dem Obturator 20 verschlossenen Zustand als normaler Schnuller verwendet werden. Dies kann dazu genutzt werden, das Kind schon vor einem medizinischen Einsatz des Saugers an diesen zu gewöhnen, z.B. vor einer Narkose. Des weiteren können spontan atmende Kinder z.B. auf Intensivstationen den Sauger in Zeiten ohne Inhalations- oder Atemtherapie behalten, was die Akzeptanz erhöht und das Bereitstellen mehrerer Schnuller überflüssig macht. Ein weiterer Vorteil einer solchen Wiederverschließbarkeit des Kanals 14 besteht darin, daß nach Ausleitung einer Narkose der Sauger ohne Trauma für das Kind einfach verschlossen werden kann, sobald der offene Kanal 14 nicht mehr benötigt wird, so daß ein mühsamer Saugeraustausch entfallen kann. Ein spezieller Vorteil des Obturators 20 im Unterschied zu einem nur mundschildseitigen, äußeren Verschließen des Kanals 14 mittels einer Folie oder eines an die Trichtermündung 15b angepaßten Stopfens besteht darin, daß sich kein Sekret, Schmutz oder dergleichen im verschlossenen Kanal 14 ansammeln kann.

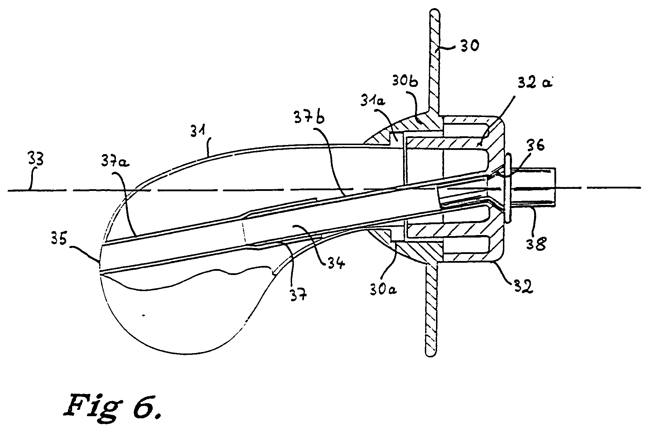

In Fig. 6 ist eine weitere Variante des Saugers der Fig. 1 und 2 mit entsprechenden vorteilhaften Funktionalitäten und medizinischen Verwendungsmöglichkeiten dargestellt. Der Sauger von Fig. 6 beinhaltet einen Mundschild 30, an dem ein sich davon nach vorn erstreckendes Mundstück 31 dadurch fixiert ist, daß es mit einem an seinem hinteren Ende vorgesehenen Ringflansch 31a gegen eine korrespondierende innere Ringschulter 30a eines hülsenförmigen Mittelbereichs 30b des Mundschilds 30 anliegt. Das Mundstück 31 ist in dieser Lage im hülsenförmigen Mittelteil 30b des Mundstücks 30 durch einen Ringfortsatz 32a einer von hinten auf den mittleren Mundschildbereich aufgesetzten Kappe 32 gesichert, die gleichzeitig als Saugergriff dient. Im Unterschied zu den vorstehend beschriebenen Beispielen besitzt das Mundstück 31 beim Sauger von Fig. 6 eine zur Längsmittenebene 33 asymmetrische, sich schräg nach unten vorwölbende Form, die besonders an den Einsatz während Narkose- und Beatmungsvorgängen angepaßt ist.

Der Sauger von Fig. 6 weist einen sich geradlinig schräg nach vorn unten verlaufenden Kanal 34 auf, der sich von einer vorderen Ausmündung 35 aus dem Mundstück 31 nach hinten durch das hohle Mundstück 31, den Mundschild 30 und die Kappe 32 hindurch in den Außenraum hinter dem Mundschild 30 erstreckt, wobei er dort auf Höhe der Längsmittelebene 33 eine mittig in der Kappe 32 vorgesehene, trichterförmige Ausmündung 36 besitzt. Der Kanal 34 ist bei diesem Beispiel von einem zweiteiligen Rohr 37 mit einer vorderen Rohrhälfte 37a und einer hinteren Rohrhälfte 37b gebildet. Die vordere Rohrhälfte 37a ist einstückig mit dem Mundstück 31 aus demselben Material gebildet, wobei sie schon aufgrund ihres gegenüber dem Mundstück 31 geringeren Durchmessers eine höhere Biegesteifigkeit besitzt als die äußere Mundstückhülle. Bei Bedarf kann zudem die Wandstärke dieser vorderen Rohrhälfte 37a größer gewählt sein als diejenige der Mundstückwandung. Die hintere Rohrhälfte 37b ist von einem mittigen Rohrfortsatz der Kappe 32 gebildet und kann je nach Wahl des Materials für die Kappe 32 biegsam oder starr ausgelegt sein. Im letzteren Fall ergibt sich die insgesamt gegebene Biegsamkeit des Kanals 34 allein durch diejenige der vorderen Rohrhälfte 37a. Die hintere Rohrhälfte 37 ist dicht und mit fluchtendem Innendurchmesser in einer korrespondierenden Aufweitung der vorderen Rohrhälfte 37a an deren hinterem Endbereich aufgenommen. Von besonderem Vorteil hinsichtlich freibleibendem Zugang zum Mund/Rachenraum ist, wie oben zur Fig. 4 erläutert, auch hier der zur Längsmittenebene 33 schräg nach vorn unten geneigte Kanalverlauf, durch den der Kanal 34 mit seiner vorderen Mündungsöffnung 35 unterhalb der Längsmittenebene 33 aus dem Mundstück 31 ausmündet.

Aus Fig. 6 ist außerdem der bereits oben erwähnte Einsatz eines Normkonnektors 38 zu erkennen, der sich durch die Wirkung der trichterförmigen Kanalmündung 36 ohne besonderen Zentrierungsaufwand von hinten in den Kanal 34 einschieben läßt. Auch im übrigen besitzt der Sauger von Fig. 6 die zu den oben beschriebenen Ausführungsbeispielen erwähnten vorteilhaften Eigenschaften und Funktionen. Es versteht sich, daß auch bei diesem Sauger die zu den anderen Beispielen erwähnten Modifikationen, wie Verwendung eines geeigneten Verschlußmittels, Vorsehen mehrerer paralleler Einzelkanäle und Wahl eines gebogenen Kanalverlaufs oder eines geradlinigen Verlaufs auf Höhe der Längsmittelebene 33 realisierbar sind.

## Patentansprüche

1. Beruhigungssauger mit
- einem Mundschild (1) und
- einem sich vom Mundschild nach vorn erstreckenden Mundstück (3),
gekennzeichnet durch
- einen biegsam und kaudruckresistent ausgelegten Kanal (6), der das Mundstück (3) und den Mundschild (1) durchquert und den Außenbereich vor dem Mundstück mit dem Außenbereich hinter dem Mundschild verbindet.

2. Beruhigungssauger nach Anspruch 1, weiter dadurch gekennzeichnet, daß der Kanal mehrere, voneinander getrennte parallele Einzelkanäle (8a, 8b) beinhaltet.

3. Beruhigungssauger nach Anspruch 1 oder 2, weiter dadurch gekennzeichnet, daß der Kanal (14) einen bezüglich einer Saugermittenebene (16) schrägen, in Saugergebrauchslage unterhalb dieser Saugermittenebene (16) am Mundstück (12) ausmündenden Verlauf aufweist.

4. Beruhigungssauger nach einem der Ansprüche 1 bis 3, weiter dadurch gekennzeichnet, daß der Kanal (14) an seinem in den Außenbereich hinter dem Mundschild (10) mündenden Ende eine trichterförmige Ausmündung (15b) aufweist.

5. Beruhigungssauger nach einem der Ansprüche 1 bis 4, weiter gekennzeichnet durch ein abnehmbares Verschlußmittel (20) für den Kanal (14).

6. Beruhigungssauger nach einem der Ansprüche 1 bis 5, weiter gekennzeichnet durch wenigstens eine seitliche Aussparung (18a, 18b) im Mundschild (10), in welcher ein Schlauch- oder Rohrelement (19) aufnehmbar ist.

7. Beruhigungssauger nach einem der Ansprüche 1 bis 6, weiter dadurch gekennzeichnet, daß der Kanal (6) von einem separaten, das Mundstück (3) und den Mundschild (1) durchquerenden, biegsamen Rohr (5) gebildet ist, das eine höhere Biegesteifigkeit als das von ihm durchquerte Mundstück (3) besitzt.
